# EUROPEAN PATENT APPLICATION

(11) **EP 4 806 999 A1**
(43) Date of publication of application: **16.09.2026**
(21) Application number: 26164167.4
(22) Date of filing: 12.03.2026
(51) Int. Cl.: C12N 15/10, C07K 14/46, G01N 33/68

(54) **PROTAMINE CONJUGATIONS FOR TRACE NUCLEIC ACID CAPTURE AND MULTI-ANALYTE RECOVERY**

(30) Priority: 13.03.2025 US 202563771209 P; 15.01.2026 US 202619449646
(71) Applicant: GE Precision Healthcare LLC, Waukesha, WI 53188 (US)
(72) Inventor: DAVIS, Brian Michael, Niskayuna 12309-1027 (US); RISHEL, Michael James, Mechanicville, 12118 (US); CHADWICK, Chrystal Mae, Niskayuna, 12309-1027 (US); NELSON, John Richard, Niskayuna, 12309-1027 (US)
(74) Representative: Kilburn & Strode LLP

(57) **Abstract**

A method for multi-analyte recovery from a single tissue sample includes lysing a tissue sample in a lysis buffer. The method also includes adding protamine conjugates (10) including protamine conjugated to a solid support matrix (14) to a lysate of the lysed tissue sample. The method further includes incubating the lysate in the presence of the protamine conjugates (10) in a binding buffer in a reaction mixture to bind nucleic acid to the protamine conjugates (10). The method even further includes removing a supernatant from the reaction mixture containing intact protein and metabolites. The method further includes eluting the nucleic acid from the protamine conjugates (10).

## Description

### CROSS-REFERENCE TO RELATED APPLICATION

This application claims priority to and the benefit of US Provisional Application No. 63/771,209, entitled "PROTAMINE CONJUGATIONS FOR TRACE NUCLEIC ACID CAPTURE AND MULTI-ANALYTE RECOVERY", filed March 13, 2025, which is herein incorporated by reference in its entirety for all purposes.

### STATEMENT REGARDING FEDERALLY SPONSORED RESEARCH & DEVELOPMENT

This invention was made with US Government support under contract number 15PNIJ-21-GG-04149-RESS awarded by National Institute of Justice (NIJ) and contract number 2018-18041000002 awarded by Intelligence Advanced Research Projects Activity (IARPA). The Government has certain rights in the invention.

### BACKGROUND

The subject matter disclosed herein relates to protamine conjugations for nucleic acid capture from biological tissues and fluids including trace samples and multi-analyte recovery.

Protamines are arginine (Arg)-rich basic proteins which replace histones during spermatogenesis and are responsible for compaction of DNA into an inactive state within the nuclei of sperm. Although most mammals contain one protamine (P1) that is responsible for packaging sperm DNA, in humans, in addition to P1, protamine P2 is present, and both are required for normal sperm function. The arginine residues in P1 protamines have been found to cluster (typically 3-7 residues) within DNA binding domains that are separated by uncharged amino acids. This is thought to allow the domains to wrap around the major groove of DNA, with the positive charge from the arginine residues neutralizing the negative charge of the phosphate groups in DNA and facilitating strong DNA binding. Mammalian P1 and P2 protamine genes are known to contain multiple cysteine residues at conserved locations. These are known to oxidize and form intra- and inter-molecular disulfide bridges within and between protamine molecules, stabilizing the chromatin complex, allowing higher levels of packaging in a series of toroids. The DNA toroids have been observed *in-vivo* as well as *in-vitro.* In contrast, fish protamine e.g., salmine from Salmon and clupeine from herring, have been found to lack cysteine residues and yet enable very tight DNA packaging. Protamine is also known to spontaneously complex with RNA and this property has been widely used to develop protamine-mRNA nanoparticles for cancer immunotherapy and use as vaccines against viruses.

Trace samples are defined as small amounts of biological tissue or biological fluids available for isolating DNA, RNA, proteins and small molecule metabolites. The small amount of biological tissue reduces the molecular yield during molecular extraction and use of state of the art molecular isolation technologies often result in low recoveries. Molecular isolation from trace samples may improve cancer biomarker detection and approaches that utilize DNA sequencing from trace samples.

State of the art methods for DNA purification use silica coated membranes or magnetic beads. Magnetic beads such as within the PrepFiler Forensic DNA Extraction Kit enables DNA purification is achieved by virtue of its ability to bind silica in the presence of high concentrations of chaotropic salts. Another type of reagent is the ChargeSwitch Forensic DNA Purification Kit, which uses magnetic beads with a unique, ionizable, nucleic acid-binding ligand whose charge can be switched based on the pH of the surrounding medium. These methods typically provide at most a 50% yield from trace samples, and none of these methods allow for co-recovery and differential elution of DNA and RNA from a single sample.

Most current commercial molecular isolation kits are designed isolate only one of either DNA, RNA, or protein, and the protocols use components (e.g., proteinase K, DNase) that are destructive to recovery of the other molecules present in the sample. Commercial kits of this type include the Qiagen QiaAmp, Qiagen RNeasy, and PicoPure extraction kits. Using these reagents, one would need to separate the initial sample into multiple fractions and independently isolate each molecule type from one of the fractions. This results in lower molecular yields from the split samples. Thus, the current available technology would require the investigator to split each trace sample to prepare multiple subsamples, one for each analyte being tested, reducing the amount of material in each test.

Multi-analyte recovery has been achieved from a single sample using commercial technology such as the Qiagen AllPrep kit for dual DNA and RNA isolation or TriplePrep kit for DNA, RNA and protein. These technologies require large amounts of input samples and they are not compatible with trace samples. Other approaches using molecular weight cut-off spin columns have been designed to isolate DNA and protease digested peptide fragments from a single sample or centrifugation of cellular material from supernatant containing extracellular DNA. It's unknown if these approaches retain recoverable RNA and they are not easily translated to high throughput automation.

Early cancer detection and early treatment results in increasingly smaller tumor sizes obtained at surgery, which requires maximizing molecular recovery from a single small sample. Traditional biobanking (i.e., storage of tissue or tumor samples) of fresh-frozen or formalin-fixed paraffin-embedded (FFPE) tissue is recovery heavy and requires dedicated staff, expertise, and storage facilities that may not be available at smaller institutes, which has the unintended impact of disenfranchising lower socioeconomic populations. Thus, there is a need for improvements in procurement technologies to address biobanking and enhance recovery of deoxyribonucleic acid (DNA), ribonucleic acid (RNA), and protein from small samples to efficiently analyze biomarkers for improved patient outcomes.

Molecular extraction from FFPE tissue is a laborious process (multiple rounds of deparaffination, heating, and chemical ex-changes) that requires specialized equipment (e.g., automated systems or acoustic sonication devices) and dedicated staff, which are not widely available (especially in lower resource settings). Additionally, fixed tissue induces DNA-protein crosslinks resulting in fragmented, reduced DNA quality for analysis. Thus, an unmet need is a simplified procurement and storage method that is easily employed in low-resource settings. Focusing the application on touch imprints would transform and innovate the process of biobanking. In an age where there are increasing demands on tissue, touch imprints only transfer cells that would be washed out during normal tissue processing, therefore there is no hesitation on the part of the procurement team to collect these specimens. There is no "loss of tissue" for diagnostic or management purposes and importantly, multiple touch imprints can be collected from the same sample, allowing analysis of tumor heterogeneity. Traditionally, DNA, RNA, and protein are individually obtained from distinct sample sections with potentially variable cellular content. Thus, DNA molecular data from one section may not correlate with protein or RNA data from a different section. The disclosed techniques would allow integrative analysis of genetic and protein data from the same cells and a broader range of tumor sizes and grades.

### BRIEF DESCRIPTION

A summary of certain embodiments disclosed herein is set forth below. It should be understood that these aspects are presented merely to provide the reader with a brief summary of these certain embodiments and that these aspects are not intended to limit the scope of this disclosure. Indeed, this disclosure may encompass a variety of aspects that may not be set forth below.

In one embodiment, a method for multi-analyte recovery from a single tissue sample is provided. The method includes lysing a tissue sample in a lysis buffer. The method also includes adding protamine conjugates including protamine conjugated to a solid support matrix to a lysate of the lysed tissue sample. The method further includes incubating the lysate in the presence of the protamine conjugates in a binding buffer in a reaction mixture to bind nucleic acid to the protamine conjugates. The method even further includes removing a supernatant from the reaction mixture containing intact protein and metabolites. The method further includes eluting the nucleic acid from the protamine conjugates.

In another embodiment, a method is provided. The method includes obtaining a tissue sample. The method also includes performing multi-analyte recovery on the tissue sample to isolate deoxyribonucleic acid, ribonucleic acid, and intact protein from the tissue sample utilizing protamine conjugates including protamine conjugated to a solid support matrix.

In a further embodiment, a method is provided. The method includes obtaining a tissue sample. The method also includes utilizing protamine conjugates including protamine conjugated to magnetic beads to recover nucleic acid from the tissue sample, wherein the nucleic acid is recovered in a quantity ranging from picograms to micrograms, and wherein the protamine conjugates are configured to bind the nucleic acid having a size ranging from 25 nucleotides to greater than 15,000 nucleotides.

### BRIEF DESCRIPTION OF THE DRAWINGS

These and other features, aspects, and advantages of the present subject matter will become better understood when the following detailed description is read with reference to the accompanying drawings in which like characters represent like parts throughout the drawings, wherein:
FIG. 1 is a schematic diagram of a conjugate (e.g., protamine conjugate) for capturing a trace amount of nucleic acid, in accordance with aspects of the present disclosure;
FIG. 2 is a schematic diagram of a workflow for multi-analyte recovery from a single tissue sample, in accordance with aspects of the present disclosure;
FIG. 3 is a flow chart of a method for trace nucleic acid capture from a small sample, in accordance with aspects of the present disclosure;
FIG. 4 is a flow chart of a method for multi-analyte recovery, in accordance with aspects of the present disclosure;
FIG. 5 is a flow chart of a method for multi-analyte recovery from a single tissue sample, in accordance with aspects of the present disclosure;
FIG. 6 depicts a graph illustrating the recovery efficiency of the disclosed conjugate to bind trace amounts of DNA, in accordance with aspects of the present disclosure;
FIG. 7 depicts an image a polyacrylamide gel depicting the ability of disclosed conjugate to bind small DNA fragments, in accordance with aspects of the present disclosure;
FIG. 8 depicts a graph illustrating the recovery efficiency of the disclosed conjugate to bind trace amounts of RNA, in accordance with aspects of the present disclosure;
FIG. 9 depicts graphs and a table illustrating DNA and RNA recovery utilizing the disclosed conjugate, in accordance with aspects of the present disclosure;
FIG. 10 depicts a graph illustrating DNA and RNA recovery from human samples and electropherograms analyzing expression of tissue-specific genes utilizing the disclosed techniques, in accordance with aspects of the present disclosure;
FIG. 11 depicts a graph illustrating DNA and RNA recovery from tumor samples utilizing the disclosed techniques, in accordance with aspects of the present disclosure;
FIG. 12 depicts a protocol for co-isolation of nucleic acids and protein from the same small sample and a table of results of utilizing the protocol, in accordance with aspects of the present disclosure;
FIG 13 depicts a protamine-conjugated swab having a bristle brush-like structure, in accordance with aspects of the present disclosure;
FIG. 14 depicts an image of an agarose gel depicting utilization of a protamine-conjugated swab for DNA binding and elution, in accordance with aspects of the present disclosure; and
FIG. 15 depicts an image of an agarose gel depicting utilization of a protamine-conjugated swab for DNA binding and elution (e.g., utilizing different protamine concentrations) and associated quantification of amount of DNA collected in accordance with aspects of the present disclosure.

### DETAILED DESCRIPTION

One or more specific embodiments will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present subject matter, the articles "a," "an," "the," and "said" are intended to mean that there are one or more of the elements. The terms "comprising," "including," and "having" are intended to be inclusive and mean that there may be additional elements other than the listed elements. Furthermore, any numerical examples in the following discussion are intended to be nonlimiting, and thus additional numerical values, ranges, and percentages are within the scope of the disclosed embodiments.

As used herein, the terms "automatic" and "automatically" refer to actions that are performed by a computing device or computing system (e.g., of one or more computing devices) without human intervention. For example, automatically performed functions may be performed by computing devices or systems based solely on data stored on and/or received by the computing devices or systems despite the fact that no human users have prompted the computing devices or systems to perform such functions. As but one nonlimiting example, the computing devices or systems may make decisions and/or initiate other functions based solely on the decisions made by the computing devices or systems, regardless of any other inputs relating to the decisions.

The present disclosure provides for a novel reagent for co-binding of trace amounts of DNA and RNA extracted from human and non-human samples. The reagent consists of protamine (a nucleic acid binder) conjugated to a solid support matrix (i.e., a protamine conjugation) such as a magnetic bead or swab. Protamine provides advantages that other proteins may not have, such as thermostability, binding characteristics to DNA and RNA, and the ability to perform in specific buffers that contain harsh chemicals. Protamine conjugations enable efficient collection and recovery of picogram (pg) to microgram (µg) quantities of nucleic acids. In contrast, conventional methods for doing the same have a poor recovery efficiency of small nucleic acid amounts. Protamine conjugations may be utilized in numerous applications including oncology for recovery of DNA and RNA in situations where there is limited patient sample (e.g., from small patient biopsies or biobanked samples). Improvements in cancer screening enable early detection of small tumors, and smaller biopsies taken that have increasingly smaller amounts of DNA, RNA, and protein for biomarker analysis. A higher recovery of these molecules would improve diagnosis from the smaller biopsies. In addition, approaches utilizing the protamine conjugations are compatible with non-destructive isolation reagents that uniquely permit recovery of DNA, RNA, and protein (intact protein) from a single trace sample (including samples consisting of less than 10,000 cells). Conventionally, only a single molecule type (DNA, RNA, or protein) would be recovered from a sample. Using a conventional approach, the original sample would need to be split into three sub-samples for individual isolation of DNA, RNA, or protein, which results in reduced recoveries of each molecule type and reducing the likelihood that a small sample would provide enough yield of any of the desired molecules. The protamine conjugations and approaches utilizing the protamine conjugations have additional applications beyond oncology (e.g., forensic analysis, biological research, genomics, gene delivery/vaccines, etc.) and may be utilized for any application where DNA, RNA, and protein are needed from small biological samples.

The disclosed embodiments enable the acquisition of a greater amount of DNA, RNA, and protein from patient samples to be used in biomarker analysis. Early detection of smaller tumors means there is less material available in biopsies. Obtaining sufficient DNA, RNA, and protein from small biopsies would benefit patients by providing more information about treatment options at an earlier time point in the progress of their disease, and potentially improving patient outcomes and saving lives. The approach is compatible with automation or manual processing, and is simple enough to be performed potentially at the patient's hospital, not requiring shipping to a specialized lab. The disclosed embodiments provide for a simplified method for multi-parametric analysis of smaller sized tumors (or tissue samples), including genomic analysis, gene expression profiling, and protein expression and activation, broadening the options for biomarker discovery as well as enabling correlative analysis of all analytes from the same sample.

The disclosed embodiments enable addressing biobanking discrepancies. The information provided from biomarkers found in biobanked tissue can guide better treatment options for patients. The disclosed embodiments provide a new lower cost approach for DNA, RNA, and protein recovery from small tissue samples to improve storage of tumor tissue and analysis for biomarkers.

The disclosed embodiments enable higher recovery of DNA, RNA, and protein from the same sample. The disclosed embodiments provides for a molecular recovery approach that would be useful for small tumor amounts including core biopsies and fine needle aspiration biopsies. The disclosed embodiments provides a method that has been validated on trace samples, with efficient recovery and elution from as little as 250 picograms of nucleic acid at low (~25 nucleotides), mid, and high (chromosomal DNA) molecular weights. In contrast, silica and anion exchange provide relatively poor recovery at low input amounts. The disclosed embodiments may enable recovery of intact proteins (e.g., complete protein with tertiary structure maintained) as opposed to denatured proteins (e.g., lacking tertiary structure and biological function) or portions of proteins. The recovery of intact proteins enables the utilization of antibody-based assays such as enzyme-linked immunosorbent assay (ELISA). The disclosed embodiments enable the utilization of targeted and untargeted assays on all three molecule types (DNA, RNA, and protein) that are more informative for discovery/diagnosis as opposed to assays on a single molecule type. In contrast, sequential slices on FFPE tissue may represent different cell types within each and therefore is not as informative as recovery of all three molecule types from a same slice with the disclosed embodiments. The disclosed embodiments enable reduced preparation time and labor for biobanking and molecular isolation via imprint preparations on glass.

The disclosed embodiments include a method for multi-analyte recovery from a single tissue sample. The method includes lysing a tissue sample in a lysis buffer. The method also includes adding protamine conjugates including protamine conjugated to a solid support matrix to a lysate of the lysed tissue sample. The method further includes incubating the lysate in the presence of the protamine conjugates in a binding buffer in a reaction mixture to bind nucleic acid to the protamine conjugates. The method even further includes removing a supernatant from the reaction mixture containing intact proteins and metabolites. The method further includes eluting the nucleic acid from the protamine conjugates.

In certain embodiments, the tissue sample includes less than 10,000 cells. In certain embodiments, the tissue sample includes a biopsy sample. In certain embodiments, the solid support matrix includes a magnetic bead, a microfluidic channel, swab, or a well plate.

With regard to the swab as a solid support matrix, touched surfaces contain extracellular nucleic acids that can be recovered by swabbing. Conjugation of protamine directly on a swab improves nucleic acid binding to the swab and facilitates nucleic acid elution. It has been demonstrated that nucleic acids bound to the swab are not eluted in traditional aqueous elution buffers, which enables washing non-nucleic acid material from the swab without loss of the nucleic acids, and subsequent elution of the nucleic acids with a reduced level of contaminants. Protamine conjugated swabs enable differential recovery of cellular material in a first fraction, followed by nucleic acid elution in a second fraction. Protamine conjugated swabs also enable a two-step collection where a standard swab collects cellular material on the surface first, and a protamine conjugated swab captures the remaining nucleic acids remaining on the surface. Further, protamine conjugated swabs enable improved nucleic acid recovery from traditionally challenging surfaces such as metals (e.g., brass fired gun casings).

In certain embodiments, the lysis buffer and the binding buffer are non-destructive to the proteins and the metabolites. In certain embodiments, eluting the nucleic acid includes eluting deoxyribonucleic acid and then eluting ribonucleic acid. In certain embodiments, eluting the nucleic acid includes eluting ribonucleic acid and then eluting deoxyribonucleic acid. In certain embodiments, eluting the nucleic acid includes co-eluting ribonucleic acid and deoxyribonucleic acid.

In certain embodiments, the protamine conjugates are configured to bind the nucleic acid with a target efficiency of 70 percent or greater. In certain embodiments, the protamine conjugates are configured to bind the nucleic acid having a size ranging from 25 nucleotides to greater than 15,000 nucleotides. In certain embodiments, eluted nucleic acid has a quantity ranging from picogram to microgram.

In disclosed embodiments, a method includes obtaining a tissue sample. The method also includes performing multi-analyte recovery on the tissue sample to isolate deoxyribonucleic acid, ribonucleic acid, and intact protein from the tissue sample utilizing protamine conjugates including protamine conjugated to a solid support matrix.

In certain embodiments, the method includes lysing the tissue sample in a lysis buffer prior to the multi-analyte recovery. In certain embodiments, performing multi-analyte recovery on the tissue sample includes adding the protamine conjugates to a lysate of the lysed tissue sample, incubating the lysate in the presence of the protamine conjugates in a binding buffer in a reaction mixture to bind nucleic acid to the protamine conjugates, and removing a supernatant from the reaction mixture containing intact proteins and metabolites, and eluting the nucleic acid from the protamine conjugates. In certain embodiments, the lysis buffer and the binding buffer are non-destructive to the proteins and the metabolites. In certain embodiments, eluted nucleic acid has a quantity ranging from picogram to microgram. In certain embodiments, the protamine conjugates are configured to bind nucleic acid having a size ranging from 25 nucleotides to greater than 15,000 nucleotides. In certain embodiments, the solid support matrix includes a magnetic bead, a microfluidic channel, swab, or a well plate.

In disclosed embodiments, a method includes obtaining a tissue sample. The method also includes utilizing protamine conjugates including protamine conjugated to magnetic beads to recover nucleic acid from the tissue sample, wherein the nucleic acid is recovered in a quantity ranging from picogram to microgram, and wherein the protamine conjugates are configured to bind the nucleic acid having a size ranging from 25 nucleotides to greater than 15,000 nucleotides.

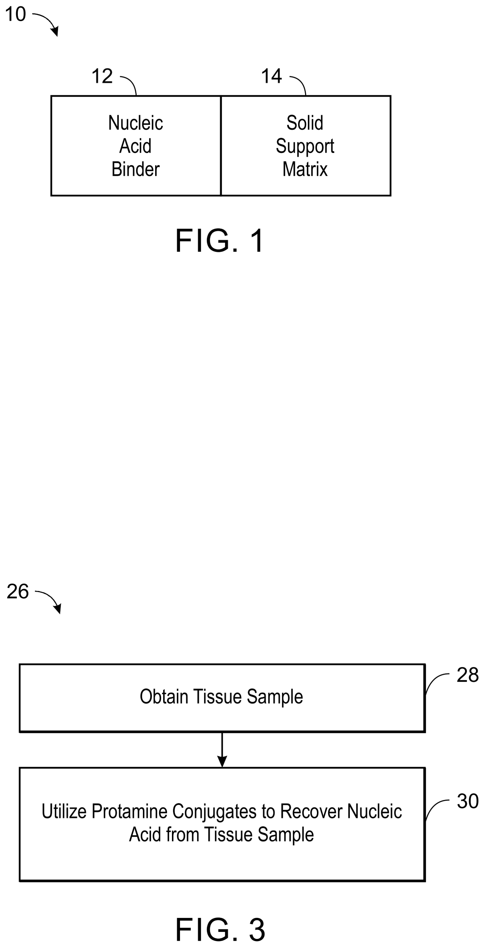

FIG. 1 is a schematic diagram of a conjugate 10 (e.g., protamine conjugate) for capturing a trace amount of nucleic acid. The conjugate 10 includes a nucleic acid binding protein or nucleic acid binder 12 coupled to a solid support matrix 14. In certain embodiments, the nucleic acid binder is protamine (e.g., a protamine lacking cysteine residues such as fish protamine). Protamines are arginine (Arg)-rich basic proteins which replace histones during spermatogenesis and are responsible for compaction of DNA into an inactive state within the nuclei of sperm. Although most mammals contain one protamine (P1) that is responsible for packaging sperm DNA, in humans, in addition to P1, protamine P2 is present, and both are required for normal sperm function. The arginine residues in P1 protamines have been found to cluster (typically 3-7 residues) within DNA binding domains that are separated by uncharged amino acids. This is thought to allow the domains to wrap around the major groove of DNA, with the positive charge from the arginine residues neutralizing the negative charge of the phosphate groups in DNA and facilitating strong DNA binding. Mammalian P1 and P2 protamine genes are known to contain multiple cysteine residues at conserved locations. These are known to oxidize and form intra- and inter-molecular disulfide bridges within and between protamine molecules, stabilizing the chromatin complex, allowing higher levels of packaging in a series of toroids. The DNA toroids have been observed *in-vivo* as well as *in-vitro.* In contrast, fish protamine e.g., salmine from Salmon and clupeine from herring, have been found to lack cysteine residues and yet enable very tight DNA packaging. Protamine is also known to spontaneously complex with RNA and this property has been widely used to develop protamine-mRNA nanoparticles for cancer immunotherapy and use as vaccines against viruses. The protamine utilized as the nucleic acid binder 12 is incredibly stable and functions over a wide variety of buffer conditions including sodium dodecyl sulfate (SDS)-denaturing and cell lysis conditions. In certain embodiments, the solid support matrix 14 is a bead such as a magnetic bead. In certain embodiments, the solid support matrix 14 is a microfluidic channel, swab, or a well plate. The solid support matrix 14 may be any surface that can bind to the nucleic acid binder 12.

The conjugate 10 can be utilized for DNA and RNA purification in a variety of sample preparation buffers that are problematic for other DNA/RNA isolation methods. For example, traditional ion exchange/silica DNA reagents are affected severely by pH and the presence of detergents. The conjugate 10 (in particular, the nucleic acid binder 12) is configured to bind to nucleic acids (both DNA and RNA) enabling greater than 95 percent binding efficiency. In addition, the conjugate 10 (in particular, the nucleic acid binder 12) is configured to bind to DNA having sizes ranging from 25 nucleotides to greater than 15,000 nucleotides with greater than 95 percent efficiency. Further, the conjugate 10 enables the co-isolation (e.g., multi-analyte recovery) of intact protein along with nucleic acids utilizing non-destructive protocols (e.g., lacking harsh chemicals). This enables obtaining analytes in buffers that are compatible with sequencing and mass spectrometry. Even further, the conjugate 10 in the disclosed methods described below enables trace amounts of nucleic acid (e.g., having a quantity ranging from picogram to microgram) to be isolated from a small tissue sample (e.g., having less than 10,000 cells). The simultaneous recovery of DNA, RNA, and protein from the same small tissue sample enables multi-parametric analysis of smaller sized tumors, including genomic analysis, gene expression profiling, and protein expression and activation, which broadens the options for biomarker discovery as well as enabling correlative analysis of all analytes from the same sample.

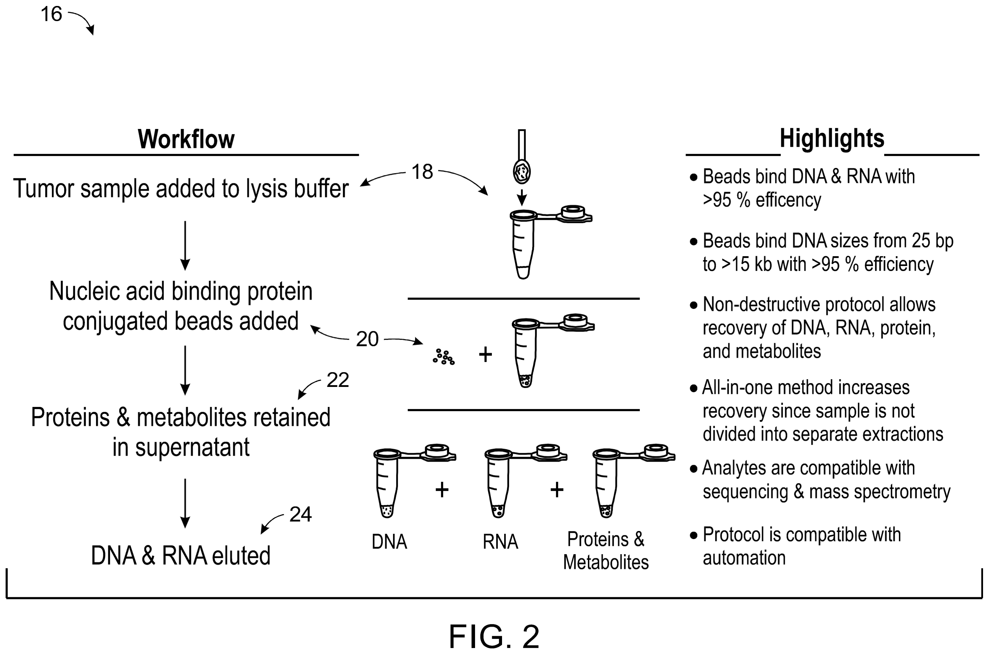

FIG. 2 is a schematic diagram of a workflow 16 for multi-analyte recovery from a single tissue sample. For example, the tissue sample may be a biopsy sample, a tumor sample, semen, blood, saliva, epithelial cells, or any other type of biological sample. Due to utilization of the conjugates 10 describe in FIG. 1, the workflow 16 is non-destructive (e.g., lacks utilizing harsh chemicals) protocol allowing the recovery of DNA, RNA, protein, and metabolites. In general, the workflow 16 includes adding a tissue sample (e.g., tumor sample) to lysis buffer as indicated by reference numeral 18. The workflow 16 also includes adding conjugates 10 to lysed tissue sample as indicated by reference numeral 20. In certain embodiments, the conjugates 10 include nucleic acid binding protein (e.g., protamine lacking cysteine residues such as fish protamine) bound to solid support matrix such as beads (e.g., magnetic beads). The workflow 16 further includes retaining intact protein and metabolites (e.g., amino acids, lipids, hormones, etc.) in a supernatant of the binding reaction between the conjugates 10 and nucleic acid as indicated by reference numeral 22. The workflow 16 further includes the elution of DNA and RNA from the conjugates as indicated by reference numeral 24. In certain embodiments, the DNA may be eluted and then the RNA eluted. In certain embodiments, the RNA may be eluted and then the DNA eluted. In certain embodiments, both the DNA and the RNA may be co-eluted.

Specifically, the workflow 16 (when utilizing a magnetic bead as the solid support matrix for the conjugates) provides a robust and reproducible for binding and elution consisting of up to a 30 minute incubation at room temperature of beads in 50 to 500 microliters (µL) of binding buffer, and then the use of a Dynamag^{™} magnet (from Thermo Fisher Scientific) to collect the nucleic acid bound beads (i.e., nucleic acid bound conjugates) for approximately 30 seconds. Subsequently, the supernatant containing unbound protein (intact protein) and metabolites is collected and the nucleic acid is eluted of the beads (i.e., the conjugates 10) using 0.5 to 1.2 molar (M) NaCl at room temperature for up to 30 minutes. The concentrations, volumes, times, and temperatures may vary from this. Other types of chemicals and concentrations may be utilized for the elution buffer.

The following provides examples of the chemicals and beads that may be used in the workflow 16. For example, magnetic Sera-Mag SpeedBeads^{™} (from Cytiva) were conjugated with the nucleic acid binding protein (e.g., protamine lacking cysteine residues such as fish protamine) to form the conjugates 10 in a method that controlled the binding density to a desired level. The beads (i.e., the conjugates 10) are stored wet in TTNA buffer (having 100 mM Tris, 150 mM NaCl, 7.7 mM NaN3, 0.1 percent (vol) Tween 20, pH 7.5) at 4 ° Celsius (C). Prior to use, the beads (i.e., the conjugates 10) are washed twice to remove excess sodium azide and detergent. The lysis buffer contains 0.4 percent sodium dodecyl sulfate (SDS) and/or 5 to 40 mM DL-dithiothreitol (DTT) or Tris(2-chloroethyl) phosphate (TCEP), and SDS sequestration buffer contains alpha-cyclodextrin concentrations sufficient to sequester SDS concentration used.. The above concentrations may vary.

The following provides example details for nucleic acid binding and elution for the workflow 16. For example, 170 to 300 µg (wet weight) of beads (i.e., conjugates 10) are added to the sample (after lysis) in a binding buffer for 30 minutes at room temperature. The lysate of the sample and the conjugates (including the binding buffer) form a reaction mixture. The tubes (having the reaction mixture) are then placed on the Dynamag^{™} magnet (i.e., the magnet) to collected the beads (i.e., conjugates 10) having the bound nucleic acid for approximately 30 seconds. While on the magnet, the tubes are slowly twisted to ensure all beads (i.e., conjugates 10 with bound nucleic acid) are bound to the magnet. The supernatant containing the unbound protein (intact protein) and metabolites are collected. The tubes are removed from the magnet, and then 50 microliters of elution buffer are added and incubated for up to 30 minutes at room temperature. In certain embodiments, the volume of the elution buffer may vary from this. The tubes are again placed on the magnet for approximately 30 seconds, and the eluted nucleic acid is recovered (via an elution buffer). Binding buffer may have 50mM Tris pH8, 150 mM NaCl, 1 mM Ethylenediaminetetraacetic acid (EDTA), and trace detergent. Elution buffer may have 0.5 M to 0.8 M NaCl. In certain embodiments, the concentration of sodium chloride may vary (e.g., up to 5 M NaCl).

The workflow 16 enables binding nucleic acids (both DNA and RNA) with greater than 95 percent efficiency. In addition, the workflow 16 enables binding DNA having sizes ranging from 25 nucleotides to greater than 15,000 nucleotides with greater than 95 percent efficiency. Further, the workflow 16 enables the co-isolation (e.g., multi-analyte recovery) of intact protein along with nucleic acids utilizing non-destructive protocols (e.g., lacking harsh chemicals). This enables obtaining analytes that are compatible with sequencing and mass spectrometry. Even further, the workflow 16 enables trace amounts of nucleic acid (e.g., having a quantity ranging from picogram to microgram) to be isolated from a small tissue sample (e.g., having less than 10,000 cells). The simultaneous recovery of DNA, RNA, and protein from the same small tissue sample enables multi-parametric analysis of smaller sized tumors, including genomic analysis, gene expression profiling, and protein expression and activation, which broadens the options for biomarker discovery as well as enabling correlative analysis of all analytes from the same sample. The workflow 16 provides an all-in-one method that increases recovery since the sample is not divided into separate extractions. The workflow 16 is compatible with automation.

FIG. 3 is a flow chart of a method 26 for trace nucleic acid capture from a small sample. In certain embodiments, steps of the method 26 may be automated. The method 26 includes obtaining a tissue sample of less than 10,000 cells (block 28). For example, the tissue sample may be a biopsy sample, a tumor sample, semen, blood, saliva, epithelial cells, or any other type of biological sample. The method 26 also includes utilizing protamine conjugates including protamine conjugated to magnetic beads to recover nucleic acid from the tissue sample with an efficiency of 95 percent or greater (block 30). The nucleic acid is recovered in a quantity ranging from picogram to microgram. The protamine conjugates are configured to bind the nucleic acid having a size ranging from 25 nucleotides to greater than 15,000 nucleotides.

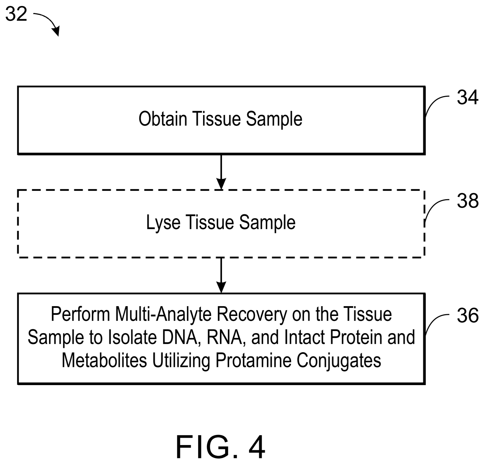

FIG. 4 is a flow chart of a method 32 for multi-analyte recovery. In certain embodiments, steps of the method 32 may be automated.

The method 32 includes obtaining a tissue sample of less than 10,000 cells (block 34). For example, the tissue sample may be a biopsy sample, a tumor sample, semen, blood, saliva, epithelial cells, or any other type of biological sample. The method 32 also includes performing multi-analyte recovery on the tissue sample to isolate deoxyribonucleic acid, ribonucleic acid, and intact protein from the tissue sample utilizing protamine conjugates including protamine conjugated to a solid support matrix (block 36).

The method 32 includes lysing the tissue sample in a lysis buffer prior to the multi-analyte recovery (block 38). In certain embodiments, performing multi-analyte recovery on the tissue sample includes adding the protamine conjugates to a lysate of the lysed tissue sample, incubating the lysate in the presence of the protamine conjugates in a binding buffer in a reaction mixture to bind nucleic acid to the protamine conjugates, and removing a supernatant from the reaction mixture containing intact proteins and metabolites, and eluting the nucleic acid from the protamine conjugates. In certain embodiments, the lysis buffer and the binding buffer are non-destructive to the proteins and the metabolites. In certain embodiments, eluted nucleic acid has a quantity ranging from picogram to microgram. In certain embodiments, the protamine conjugates are configured to bind nucleic acid with an efficiency of 95 percent or greater. In certain embodiments, the protamine conjugates are configured to bind nucleic acid having a size ranging from 25 nucleotides to greater than 15,000 nucleotides. In certain embodiments, the solid support matrix includes a magnetic bead, a microfluidic channel, swab, or a well plate.

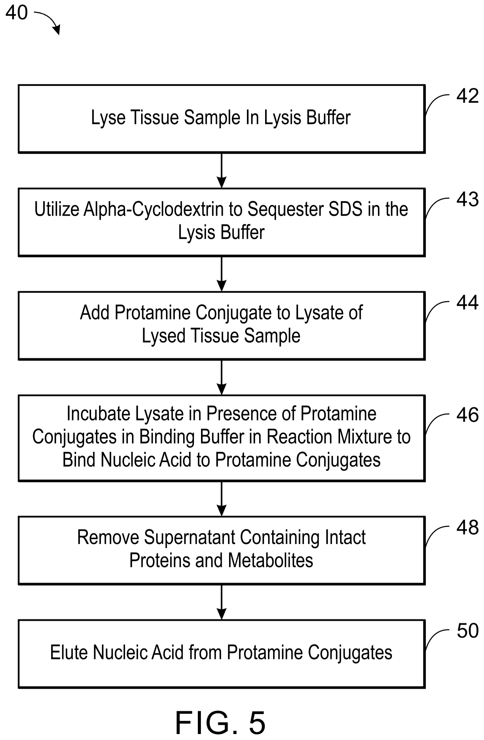

FIG. 5 is a flow chart of a method 40 for multi-analyte recovery from a single tissue sample. In certain embodiments, steps of the method 40 may be automated.

The method 40 includes lysing a tissue sample in a lysis buffer (block 42). In certain embodiments, the method 40 includes, prior to incubating the lysate in the presence of the protamine conjugates, utilizing alpha-cyclodextrin (as part of a sequestration buffer) to sequester sodium dodecyl sulfate in the lysis buffer to enhance binding of nucleic acid to the protamine conjugates (block 43). The method 40 also includes adding protamine conjugates including protamine conjugated to a solid support matrix to a lysate of the lysed tissue sample (block 44). The method 40 further includes incubating the lysate in the presence of the protamine conjugates in a binding buffer in a reaction mixture to bind nucleic acid to the protamine conjugates (block 46). The method 40 even further includes removing a supernatant from the reaction mixture containing intact proteins and metabolites (block 48). The method 40 further includes eluting the nucleic acid from the protamine conjugates (block 50).

In certain embodiments, the tissue sample includes less than 10,000 cells. In certain embodiments, the tissue sample includes a biopsy sample. In certain embodiments, wherein the solid support matrix includes a magnetic bead, a microfluidic channel, swab, or a well plate.

In certain embodiments, the lysis buffer and the binding buffer are non-destructive to the proteins and the metabolites. In certain embodiments, eluting the nucleic acid includes eluting deoxyribonucleic acid and then eluting ribonucleic acid. In certain embodiments, eluting the nucleic acid includes eluting ribonucleic acid and then eluting deoxyribonucleic acid. In certain embodiments, eluting the nucleic acid includes co-eluting ribonucleic acid and deoxyribonucleic acid.

In certain embodiments, the protamine conjugates are configured to bind the nucleic acid with an efficiency of 95 percent or greater. In certain embodiments, the protamine conjugates are configured to bind the nucleic acid having a size ranging from 25 nucleotides to greater than 15,000 nucleotides. In certain embodiments, eluted nucleic acid has a quantity ranging from picograms to micrograms.

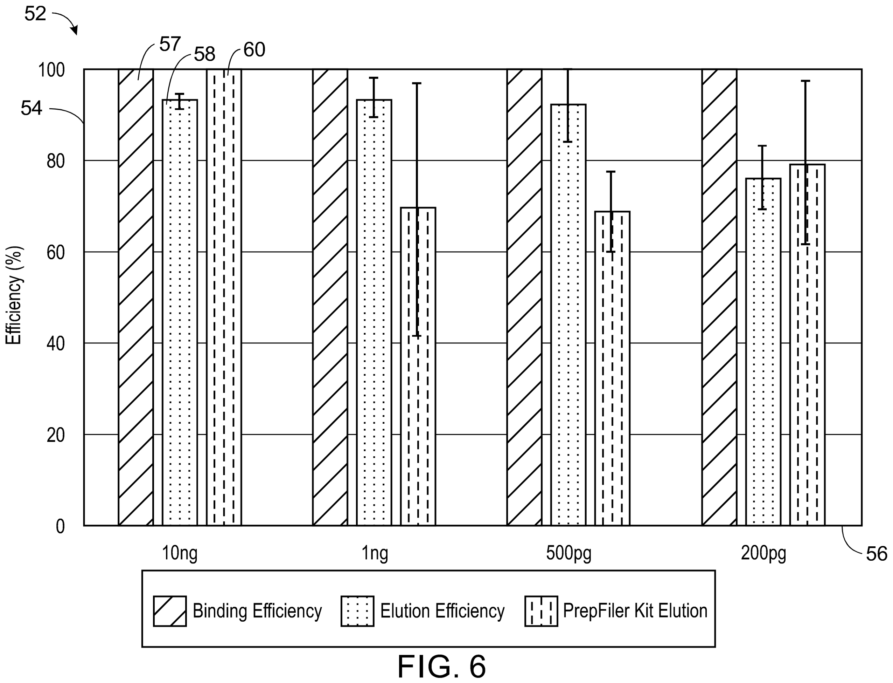

FIG. 6 depicts a graph 52 illustrating the recovery efficiency of the disclosed conjugate (e.g., utilizing protamine conjugates having protamine conjugated to a solid support matrix (e.g., magnetic bead)) to bind trace amounts of DNA. The graph 52 includes a y-axis 54 representing efficiency and an x-axis 56 representing a starting amount of inputted DNA (e.g., 10 ng, 1 ng, 500 pg, and 200 pg). Bars 57 represent binding efficiency at the different starting amounts of inputted DNA. Bars 58 represent elution efficiency at the different starting amounts of inputted DNA. Bars 60 represent elution efficiency utilizing a DNA extraction kit that utilizes harsh chemicals (e.g., PrepFiler^{™} Forensic DNA Extraction Kit from Thermo Fisher Scientific).

The recovery efficiency of 10 to 100 ng input DNA starting amounts (comparable to ~2000 to ~20,000 cells) was similar to 500 pg trace DNA recovery. As depicted in the graph 52, the disclosed techniques compared to the DNA extraction kit that utilizes harsh chemicals, were found to have comparable or better elution, and more consistent performance. For example, the protamine conjugates having protamine conjugated to a solid support matrix (e.g., magnetic beads) had greater than 90 percent elution efficiency at input amounts as low as 500 pg DNA, while the DNA extraction kit that utilizes harsh chemicals had 70 to 80 percent elution efficiency (and was more inconsistent). The initial and eluted DNA was run on an agarose gel to assess any bias in binding/elution of specific sizes of DNA, and no bias was found. DNA as small as 25 bp, and as large as the limit of detection of the agarose gel (>15 kb) was efficiently bound and eluted. Thus, the protamine conjugates having protamine conjugated to a solid support matrix (e.g., magnetic beads) performed as well as commercial DNA extraction kits that utilize harsh chemicals.

Highly degraded genomic DNA samples, an average size of 150 bp (ranging between 25 to 500 bp), were generated to serve as a surrogate for biobanked samples in fixatives and drug treated tumor samples. The Quantifiler^{™} Trio degradation index value was approximately 5.5 for the sheared DNA. Using 0.8M NaCl as the elution buffer, the protamine conjugates having protamine conjugated to a solid support matrix (e.g., magnetic beads) performed similarly at degraded DNA concentrations down to 200 pg with recoveries of approximately 60 percent to 90 percent.

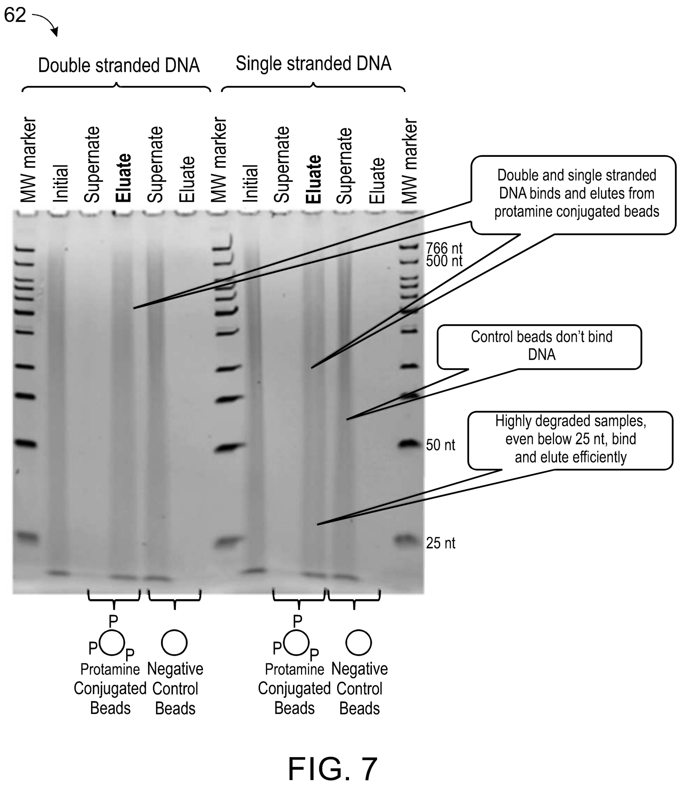

FIG. 7 depicts an image 62 of a polyacrylamide gel depicting the ability of disclosed conjugate (i.e., the protamine conjugates having protamine conjugated to a solid support matrix (e.g., magnetic beads)) to bind small DNA fragments. DNA was sheared to 20-800 bp in size to mimic severely damaged DNA caused by chemotherapeutic drug treatment/fixatives in biobanked samples. The DNA was denatured to single strands by heating to 95 °C or kept double stranded, then protein conjugated beads or non-conjugated negative control beads were added. All sizes of DNA bound efficiently, as depicted by the lack of DNA in the supernatant and presence of all sizes of DNA in the eluted fraction including 20-100 bp and 20-100 nucleotide fragments. This is a significant finding because recovery of very small DNA fragments using traditional ion exchange resins and silica beads is very inefficient.

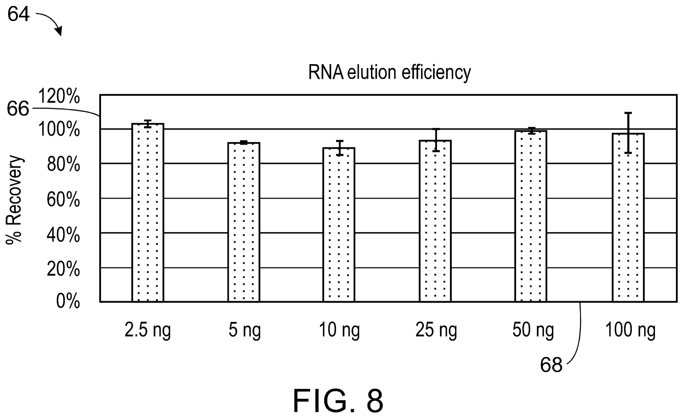

FIG. 8 depicts a graph 64 illustrating the recovery efficiency of the disclosed conjugate (i.e., the protamine conjugates having protamine conjugated to a solid support matrix (e.g., magnetic beads)) to bind trace amounts of RNA. The graph 64 includes a y-axis 66 representing percent recovery and an x-axis 68 representing a starting amount of inputted RNA (e.g., 2.5 ng, 5 ng, 10 ng, 25 ng, 50 ng, and 100 ng). Bars 70 represent recovery efficiency at the different starting amounts of inputted RNA. To develop protocols for RNA binding and elution, 2.5 ng to 100 ng of human spleen total RNA were used. Efficient RNA binding and elution was achieved using the same binding buffer and 0.8M NaCl elution buffer. The eluted RNA was quantified using the Quant-iT^{™} RiboGreen RNA assay (from Thermo Fisher Scientific). The disclosed conjugate provided consistent very high levels of binding (on average > 95 percent) and elution (on average > 89 percent), with many samples eluting at >95 percent efficiency in 0.8M NaCl. Thus, the protamine conjugates having protamine conjugated to a solid support matrix (e.g., magnetic bead) recover both DNA and RNA using the same buffers and binding/elution conditions.

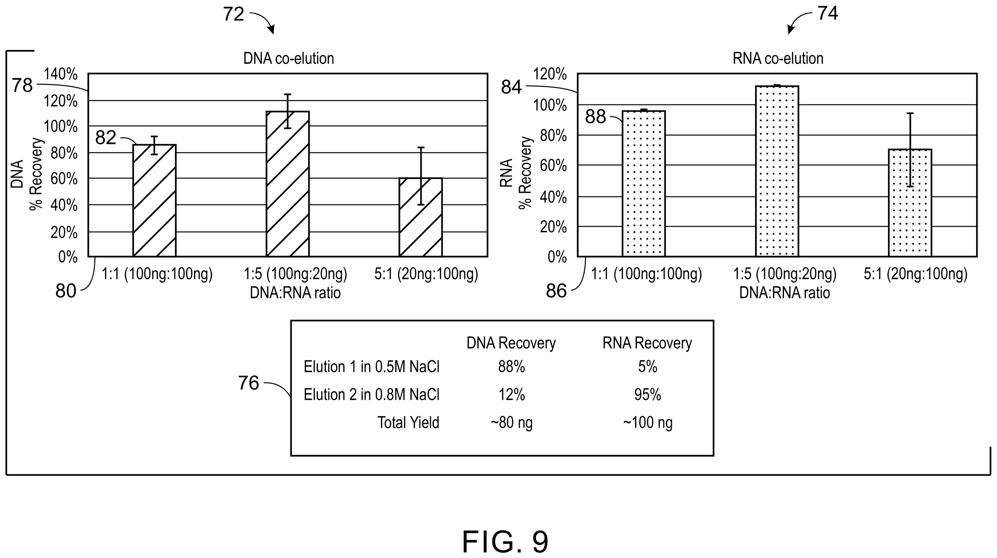

FIG. 9 depicts graphs 72, 74 and a table 76 illustrating DNA and RNA recovery utilizing the disclosed conjugate (i.e., the protamine conjugates having protamine conjugated to a solid support matrix (e.g., magnetic beads)). Co-binding and co-elution of genomic DNA and total RNA within the same input sample was assessed using a single binding and elution condition in FIG. 9. Samples tested included a mix of DNA and RNA at 1:1, 5:1, and 1:5 ratios (comprising 20 to 100 ng input amounts). To eliminate DNA/RNA cross-detection using PicoGreen^{™} and RiboGreen^{™}, eluted samples were digested with 1U of RNase A prior to DNA quantification, or 1U of DNase I prior to RNA quantification.

Graph 72 depicts DNA co-elution. Graph 72 includes a y-axis 78 representing percent recovery and an x-axis 80 representing the different ratios of DNA to RNA. Bars 82 represent recovery efficiency of DNA at the different ratios of DNA to RNA. Graph 74 depicts RNA co-elution. Graph 74 includes a y-axis 84 representing percent recovery and an x-axis 86 representing the different ratios of DNA to RNA. Bars 88 represent recovery efficiency of RNA at the different ratios of DNA to RNA. Mixed DNA and RNA samples were recovered at similar efficiency as the individual nucleic acid control samples as depicted in the graphs 72, 74. Thus, these data confirm that DNA and RNA can be co-bound and co-eluted from the same sample with recoveries consistent with prior single molecule inputs.

Differential recovery of DNA and RNA may be advantageous compared to existing technology workflows in which trace samples would need to be split into two fractions, each containing half the initial amount of DNA and RNA. Modulating the salt concentrations in elution buffers promoted stepwise elution of DNA followed by RNA, but the differential samples were not pure. The greatest differential between DNA and RNA elution was observed at 0.5M NaCl vs 0.8M NaCl. 0.5M NaCl was sufficient to elute approximately 80% of the bound DNA, with minor RNA contamination (about 5 percent of the total RNA) as depicted in the table 76. It should be noted that other concentrations of sodium chloride can be utilized to enable differential RNA and DNA elution. Likewise, 0.8M contained about 95 percent of the RNA with approximately 10-15 percent of the total DNA eluting in the 0.8M NaCl fraction as depicted in the table 76. Thus, a trade-off of differential elution is a reduced total yield relative to co-elution. Differential elution separates and provides greater than 80 percent yield of each nucleic acid type into two separate fractions, compared to 50 percent of each nucleic acid type recovered in each fraction if the sample was simply split into two.

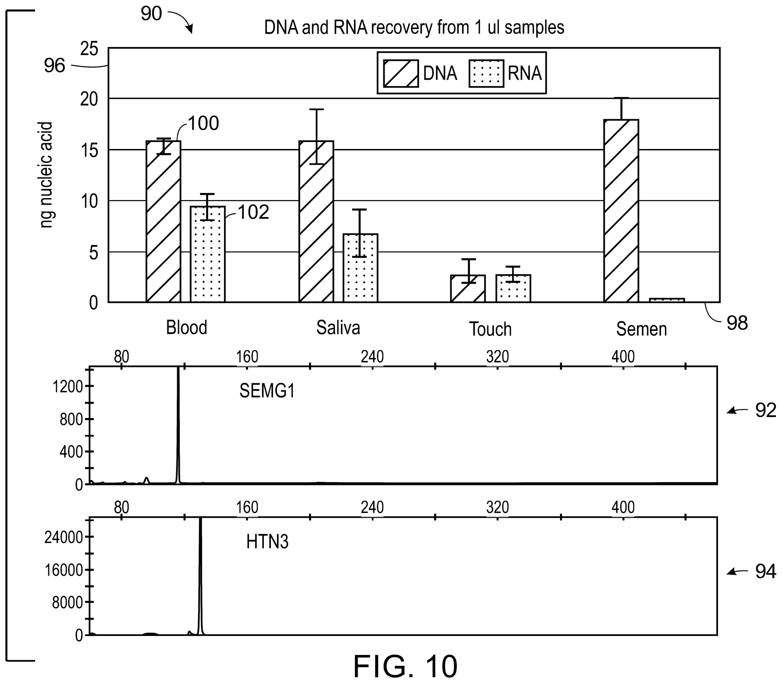

FIG. 10 depicts a graph 90 illustrating DNA and RNA recovery from human samples (utilizing the disclosed conjugate (i.e., the protamine conjugates having protamine conjugated to a solid support matrix (e.g., magnetic beads))) and electropherograms 92, 94 analyzing expression of tissue-specific genes utilizing the disclosed techniques. Blood, saliva, semen and finger touches were tested for recovery of DNA and RNA leveraging the protocols developed above. Blood and saliva were lysed in buffers containing 0.4 percent SDS with or without 5mM DTT. Semen was lysed in buffers containing 0.4 percent SDS and 40mM DTT. Touch samples were lysed in buffers containing 5 to 10 mM DTT. The SDS tolerance of the nucleic acid binding protocol is approximately 0.0025 percent to 0.005 percent final concentration, therefore a method to sequester SDS was developed using alpha-cyclodextrin (ACD). ACD is a cyclic oligosaccharide that has specificity to SDS, sequestering it in solution and restoring function to SDS sensitive enzymatic reactions such as PCR. ACD effectively neutralizes 0.4 percent SDS lysis buffers at a 1:1 molar ratio, and enables DNA and RNA binding to the magnetic beads.

The nucleic acid binding protein conjugated to the solid support matrix lacks cysteine amino acids, and therefore should be relatively resistant to reducing agents. Reducing agents help facilitate lysis of certain case type samples with cell membranes that contain an abundance of crosslinked proteins (such as cornified epithelial cells and semen). Efficient DNA and RNA binding was achieved at 2 mM DTT concentrations, and in some samples efficient nucleic acid recovery was obtained from samples containing up to 10 mM DTT.

To assess co-recovery of DNA and RNA, the DNA content was quantified using PicoGreen^{™} and Quantifiler^{™} Trio, and RNA was quantified using RiboGreen^{™}. Graph 90 includes a y-axis 96 representing an amount of nucleic acid recovered and an x-axis 98 representing the type of human sample. Bars 100 represent the amount of DNA. Bars 102 represent the amount of RNA. As depicted in the graph 90, high DNA and RNA recovery was obtained from blood, saliva, fingerprints, and semen. From 1 µl of blood, approximately 15 to 20 ng of DNA was obtained. From 1 µl of saliva approximately 15 ng of DNA was recovered. From 2 µl of semen, approximately 18 ng of DNA was recovered. RNA yields of 9.4 ng from 1 µl of blood, 6.8 ng from 1 µl of saliva, and 2.8 ng from fingerprints were recovered. RT-PCR followed by capillary electrophoresis confirmed expression of the tissue-specific genes SEMG1 from semen and HTN3 from saliva as depicted in the electropherograms 92 and 94, respectively.

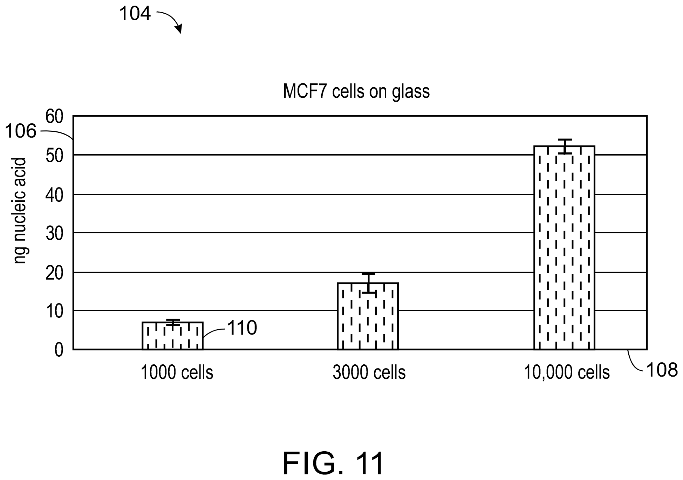

FIG. 11 depicts a graph 104 illustrating DNA and RNA recovery from tumor samples utilizing the disclosed techniques (utilizing the disclosed conjugate (i.e., the protamine conjugates having protamine conjugated to a solid support matrix (e.g., magnetic beads))). Graph 104 includes a y-axis 106 representing an amount of nucleic acid recovered and an x-axis 108 representing an amount of cells of the tumor sample. Bars 110 represent the amount of nucleic acid recovered for the different amounts of cells. DNA and RNA recovery on tumors samples was confirmed using the MCF7 breast cancer cell line, a surrogate for the cellular content within primary tumor touch imprints. Cells were deposited onto glass slides at either 1000, 3000, or 10,000 cells per slide (for an expected approximately 6 ng DNA, approximately 18 ng DNA, approximately 60 ng DNA, respectively), then air dried. In this preliminary experiment, a lysis, binding and elution protocol designed for blood extraction was used and nucleic acid content was assessed by PicoGreen^{™} and RiboGreen^{™}. The results depicted in the graph 104 demonstrate efficient recovery from the MCF7 cell line, aligned with the expected yields above.

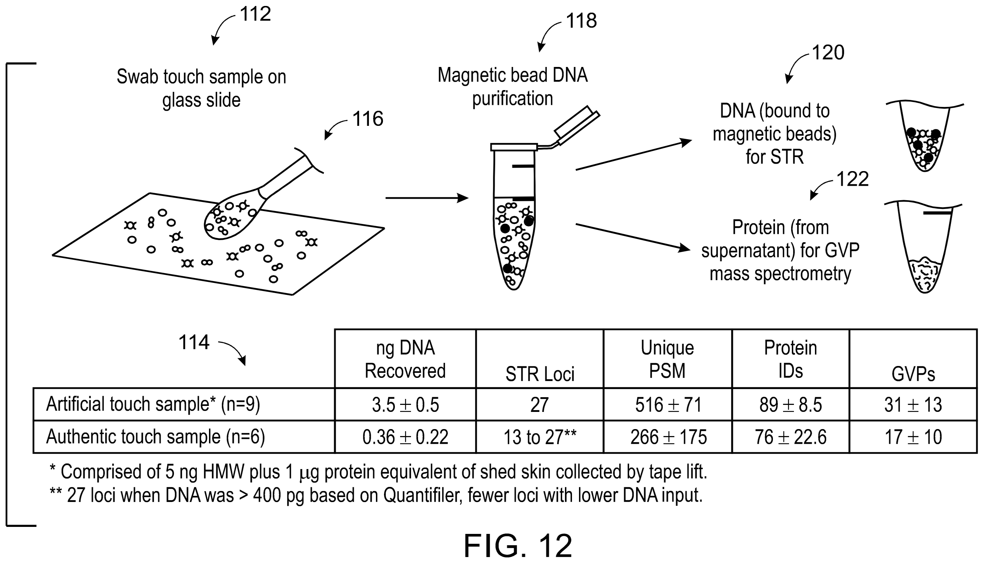

FIG. 12 depicts a protocol 112 (utilizing the disclosed conjugate (i.e., the protamine conjugates having protamine conjugated to a solid support matrix (e.g., magnetic beads))) for co-isolation of nucleic acids and proteins from the same small sample and a table 114 of results of utilizing the protocol 112. Differential DNA and protein recovery in artificial (~10,000 shed skin cells that are known to have very low DNA content seeded onto a glass slide with artificial sebum as depicted by reference numeral 116 of the illustrated protocol 112) and authentic fingerprint (index finger) touch samples were assessed. Magnetic bead DNA purification was conducted utilizing the disclosed conjugate as indicated by reference numeral 118 of the illustrated protocol 112. DNA was recovered for short tandem repeat (STR) polymerase chain reaction (PCR) analysis as indicated by reference numeral 120 of the illustrated protocol 112. Protein was recovered for genetically variant peptide (GVP) mass spectrometry as indicated by reference numeral 122 of the illustrated protocol 112. DNA recoveries from the magnetic beads were approximately 3.5 ng from the artificial samples and about one tenth of that from the authentic samples as depicted in the table 114. The fraction that remained unbound to the magnetic beads contained 2 to 10 µg of protein as quantified by Micro-BCA^{™} Protein Assay Kit (from Thermo Fisher Scientific). A normal distribution within the protein banding pattern was observed after polyacrylamide gel electrophoresis and silver staining. The protein was subjected to trypsin digest and iodoacetamide alkylation prior to analysis by mass spectrometry, where the protein fraction from finger-prints provided >250 unique peptide spectrum matches (PSMs) and >75 protein IDs. Donor-specific genetically variant peptides (GVPs) were quantified based on an internal derived GVP atlas found predominantly within keratin subtypes and hornerin, demonstrating that human identification was achievable by bioinformatic analysis of the peptides and DNA sequence. Note that the procedure does not use proteases, therefore the protein recovered is intact and not digested into peptides. This potentially valuable feature enables analysis using antibody based downstream assays while remaining compatible with trypsinization prior to mass spectrometry.

Purchased single donor semen (Innovative Research, Novi, MI, USA) and freshly collected saliva and blood from volunteers were used. Body fluids were spotted onto new glass microscope slides cleaned with ethanol in 1 µL volumes (semen, blood) or 2 µL (saliva) and allowed to air dry. Dried samples were collected using FLOQSwabs^{®} moistened with 2 µL of lysis buffer (0.4% SDS, 10 mM Tris HCl, 1 mM EDTA, with or without 40 mM DTT or 5 to 50 mM TCEP).

Swab tips were broken into tubes containing ~50 to 200 µL of lysis buffer. Tubes were placed in a thermomixer (Eppendorf, Hamburg, Germany) for 30 min at 900 rpm. The lysate was recovered from the swabs through centrifugation using Investigator Lyse&Spin baskets (Qiagen, Hilden, Germany). Alpha cyclodextrin (ACD) was then added to a 1:1 or 2:1 molar ratio (α-cyclodextrin:SDS) and tubes were incubated at room temperature for 10 min. Protamine conjugated beads (300 µg per sample) were added to the lysate in TNT buffer (50 mM Tris HCl, 150 mM NaCl, 1 mM EDTA, trace detergent) Samples were placed in the thermomixer at 900 rpm for 30 minutes at room temperature for binding of nucleic acids to protamine. Samples were placed on a magnetic stand for one minute and the supernatant was collected and saved for protein analysis. Elution of bound nucleic acids was performed using 50 µL of 0.5M to 1.2 M NaCl, incubating at room temperature for 30 minutes and placing on a magnetic stand for recovery of eluate. Eluted nucleic acids were purified using Microcon^{®} DNA Fast Flow centrifugal filters (MilliporeSigma, Burlington, MA, USA), After washing, nucleic acid recovery was performed using 0.0001X TE buffer added to the filter, inverted, placed in a new 1.5 mL tube and centrifuged at 1000 g for 3 minutes.

RNA was used to amplify body fluid specific markers. One marker each for semen (Semenogelin1, SEMG1), saliva (Histatin3, HTN3), and blood (5-AminoLevulinate Synthase 2, ALAS2) was selected for amplification. Complementary DNA was synthesized using half reactions of the LunaScript RT SuperMix Kit (New England BioLabs) according to manufacturer's instructions using 5 µL of nucleic acid extract. A 2 µL aliquot of the cDNA product was then amplified using Platinum II Hot-Start PCR Master Mix (Thermo Fisher Scientific)..

PCR was performed in 10 µL with a final concentration of 0.25 µM of fluorescently labeled forward and unlabeled reverse primers using the following cycling conditions (94 °C for 2 minutes followed by 32 cycles of 94 °C for 30 s, annealing temperature for 30 s and 72 °C for 30 s, and a final extension step at 72 °C for 10 minutes. The Annealing temperature used was 50 °C for SEMG1, 56 °C for ALAS2, and 55 °C for HTN3. Forward primers were labeled with 6-FAMTM dye for SEMG1 and VICTM dye for ALAS2 and HTN3. Amplified products were analyzed using CE on an Applied Biosystems 3500 Genetic Analyzer using the GeneScanTM 600 LIZTM dye Size Standard (Thermo Fisher Scientific) and POP-4TM polymer. Results were viewed using GeneMapper^{®} ID-X v1.4 (Applied Biosystems).

Supernatant fractions from 6 extractions (2 semen, 2 saliva, and 2 blood) were analyzed using a Fusion Lumos Orbitrap mass spectrometer (ThermoFisher, Massachusetts, USA). Both full MS scans and MS2 scans were acquired in orbitrap mass analyzer. MS2 data were acquired using HCD fragmentation methods. All raw data files were used for data analysis using the PEAKS Studio 12 software. A database search was performed against the Homo sapiens database, and a 1% false discovery rate (FDR) cut-off was applied. Carbamidomethylation was set as a fixed modification, with N-acetylation and oxidation set as variable modifications. Supernatant fractions from 6 extractions (2 semen, 2 saliva, and 2 blood) resulted in successful identification of ≥346 proteins in all samples.

There is currently no commercial kit for co-extraction of DNA and RNA from trace biological samples. Kits that allow co-extraction of DNA and RNA have been found to be highly variable in effectiveness and completely unsuitable for trace samples. The method of the invention offers efficient co-recovery of DNA and RNA from biological samples which can be used for PCR and sequencing, RNA sequencing, and SNP detection, as well as recovery of proteins which can be used for biomarker analysis, mass spectrometry and ELISA.

Molecular extraction from FFPE tissue is a laborious process (multiple rounds of deparaffination, heating, and chemical ex-changes) that requires specialized equipment (e.g., automated systems or acoustic sonication devices) and dedicated staff, which are not widely available (especially in resource-limited situations). Additionally, fixed tissue induces DNA-protein crosslinks resulting in fragmented, reduced DNA quality for analysis. Thus, an unmet need is a simplified procurement and storage method that is easily employed in low-resource settings. Focusing the application on touch imprints would transform and innovate the process of biobanking. In an age where there are increasing demands on tissue, touch imprints only transfer cells that would be washed out during normal tissue processing, therefore there is no hesitation on the part of the procurement team to collect these specimens. There is no "loss of tissue" for diagnostic or management purposes and importantly, multiple touch imprints can be collected from the same sample, allowing analysis of tumor heterogeneity. Traditionally, DNA, RNA, and protein are individually obtained from distinct sample sections with potentially variable cellular content. Thus, DNA molecular data from one section may not correlate with protein or RNA data from a different section. The disclosed techniques would allow integrative analysis of genetic and protein data from the same cells and a broader range of tumor sizes and grades.

The disclosed techniques provide a substantial improvement and transformative potential. The disclosed techniques enable multi-analyte recovery from easy-to-generate, standard touch imprints from newly excised tumors, first focusing on lung, breast and colorectal cancer, but the possibility to extend to other cancers in the future. The touch imprints can be performed by surgical staff, requiring no additional personnel. This approach contrasts with the traditional methods that require dedicated on-call staff, liquid nitrogen, and other specialized resources. The need for these resource-intensive methods makes it impossible to collect specimens in smaller resource-poor institutions and has led to elitism in facilities that can afford to provide biobanking services. The disclosed techniques would provide multi-parametric analysis from small amounts of tissue, and the capability to do genomic analysis for mutations, deletions and translocations, tumor gene expression profiling, and quantifying protein expression and activation-expanding the range of biomarker reference data from a broader representation of biobanks and addressing the current socio-economic related disparities in biobanking. In addition, multianalyte analysis from trace samples could provide novel insights into the biology of metastatic disease. The transformative potential of our proposed approach is to democratize biobanking practices using simplified methods for touch imprint biobanking paired with simultaneous recovery of DNA, RNA, and protein from a single sample section, which can be performed at low-resource locations.

As mentioned above, the solid support matrix may be a swab. Protamine conjugated swabs facilitate the collection of extracellular touch sample DNA, including off hard-to-recover surfaces such as brass, due to the specific chemical interaction that takes place between nucleic acid and protamine. In one embodiment, swabs are conjugated by modifying the structure of the nylon material used in FLOQSwabs^{®}, and covalently conjugating protamine onto the modified nylon. In one embodiment, protamine conjugated swabs bind nucleic acids deposited onto the swab. In another embodiment, protamine conjugated swabs bind nucleic acids on swabbed surfaces. In another embodiment, the surfaces contain mixtures of extracellular DNA and bodily fluids. In another embodiment, the surfaces contain mixtures of extracellular DNA and cells. Surfaces can be of any material including, but not limited to, glass, metal, plastic, stone, pavement, brick and cloth.

Protamine conjugated swabs bind strongly to nucleic acids and the nucleic acids remain bound to the swabs during traditional wash buffers and conditions. Elution occurs under conditions of high ionic strength. Protamine swabs exhibit similar performance for binding and elution as protamine conjugated onto magnetic beads.

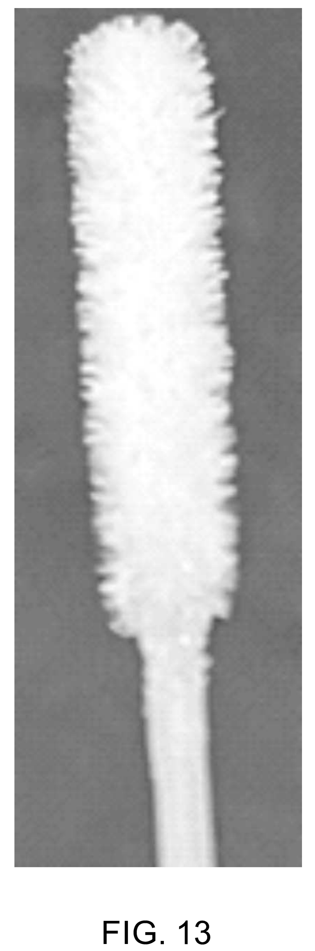

In one embodiment, protamine conjugated swabs increase liquid absorbency, which is released by gentle low-speed centrifugation. In another embodiment, the protamine swabs gain a "bristle brush"-like structure as depicted in FIG. 13. In another embodiment, protamine swabs can be stored dried with no loss in potency.

In one example, proof of concept for DNA binding and elution was done by pipetting 100 ng of DNA onto the swab and eluting in buffers of increasing NaCl concentration. Human genomic DNA (Promega) was sheared using a g tube (Covaris) to average size of 6 to 10 kb, as a surrogate for touch sample DNA. The DNA was added to the swab either in TE buffer, or in our 1x TNT binding buffer (50mM Tris pH 8, 150mM NaCl, 1mM EDTA, 0.01% Tween-20), with comparable results for TE and TNT.

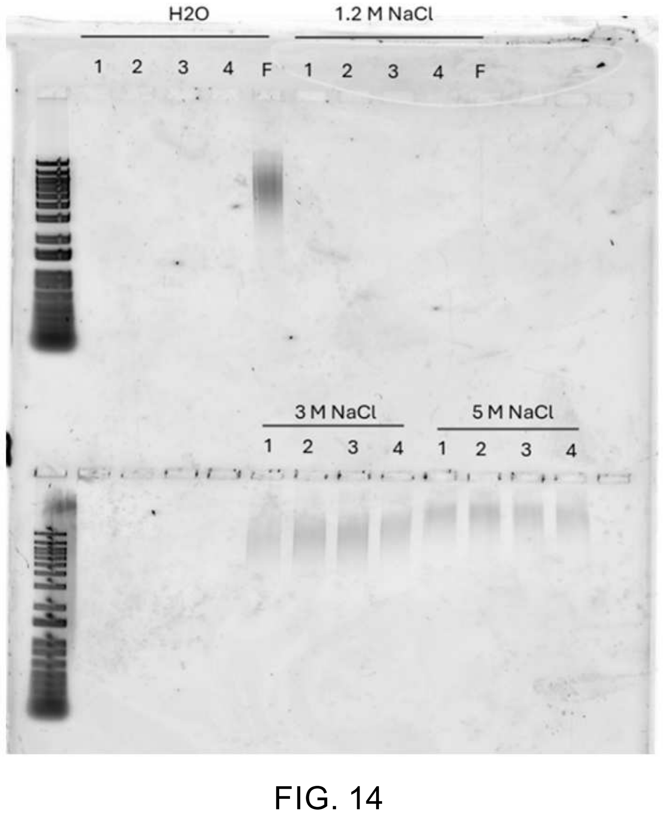

The results of DNA elution from protamine conjugated swabs and unmodified FLOQ swabs are shown in FIG. 14, using a series of buffers with increasing ionic strength. The swabs were successively washed in water, then 1.2M NaCl, then 3 M NaCl and finally 5 M NaCl. As shown in FIG. 14, the DNA was removed from the FLOQSwab^{®} in a water wash, while elution off protamine swabs required a higher ionic strength buffer. In the example shown, a buffer of greater than 1.2M NaCl was required for elution from the protamine conjugated swab but alternative strategies for reduced protamine load reduces the ionic strength needed for the elution buffer. The reduced ionic strength buffer still enables differential elution of nucleic acids from proteins, lipids and metabolites, but uses buffer compositions that are more compatible with downstream reaction conditions used for nucleic acid amplification and analysis.

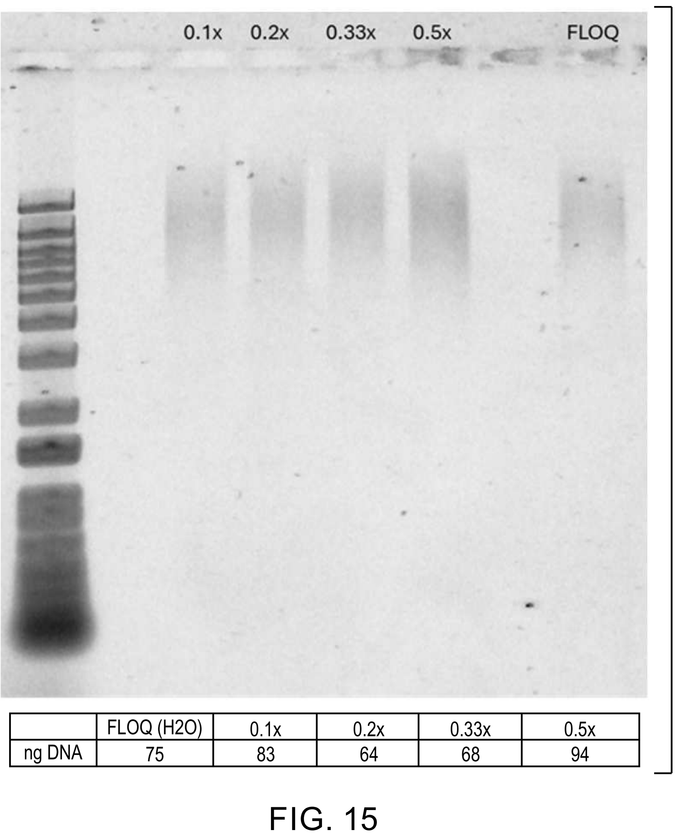

In another example, protamine loaded at 0.1x the amount used in the initial experiments described above resulted in similar binding and elution at the same NaCl concentrations as the swabs loaded with higher protamine amounts. FIG. 15 shows the results of protamine swab collections of 100 ng DNA deposited onto glass slides, analyzed by gel electrophoresis and PicoGreen^{™} for quantification. The amount of DNA collected using protamine swabs and eluted in NaCl was comparable to standard FLOQSwabs^{®} eluted in water, showing similar recovery efficiency.

In another example, protamine swabs can be used to recover nucleic acid directly from a bodily fluid or cellular material, bypassing the need for protamine beads. In one embodiment, the bodily fluids and cellular material are exposed to a lysis buffer, then nucleic acids are bound to the protamine swab and differentially eluted from the other cellular molecular material.

Technical effects of the disclosed embodiments include providing for a novel reagent for co-binding of trace amounts of DNA and RNA extracted from human and non-human samples. Technical effects of the disclosed embodiments include increasing sensitivity of biomarker assays by providing greater amounts of DNA, RNA, and protein from small-sized patient samples such as biopsies and biobanked samples including touch imprints. Technical effects of the disclosed embodiments include providing a fast and inexpensive approach that could be automated and that could be utilized in hospitals at the point of care (not just in centralized facilities). Technical effects of the disclosed embodiments include enabling recovery of biomarkers from early stage/early detected tumors toward personalized treatments and better patient outcomes.

The techniques presented and claimed herein are referenced and applied to material objects and concrete examples of a practical nature that demonstrably improve the present technical field and, as such, are not abstract, intangible or purely theoretical. Further, if any claims appended to the end of this specification contain one or more elements designated as "means for [perform]ing [a function]..." or "step for [perform]ing [a function]...", it is intended that such elements are to be interpreted under 35 U.S.C. 112(f). However, for any claims containing elements designated in any other manner, it is intended that such elements are not to be interpreted under 35 U.S.C. 112(f).

This written description uses examples to disclose the present subject matter, including the best mode, and also to enable any person skilled in the art to practice the subject matter, including making and using any devices or systems and performing any incorporated methods. The patentable scope of the subject matter is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A method for multi-analyte recovery from a single tissue sample, comprising:
lysing a tissue sample in a lysis buffer;
adding protamine conjugates (10) comprising protamine conjugated to a solid support matrix (14) to a lysate of the lysed tissue sample;
incubating the lysate in the presence of the protamine conjugates (10) in a binding buffer in a reaction mixture to bind nucleic acid to the protamine conjugates (10);
removing a supernatant from the reaction mixture containing intact protein and metabolites; and
eluting the nucleic acid from the protamine conjugates (10).

2. The method of claim 1, further comprising, prior to incubating the lysate in the presence of the protamine conjugates (10), utilizing alpha-cyclodextrin to sequester sodium dodecyl sulfate in the lysis buffer to enhance binding of nucleic acid to the protamine conjugates (10).

3. The method of claim 1, wherein the tissue sample comprises a biopsy sample, a touch imprint, human cells, non-human cells, blood, plasma, semen, saliva, or epithelial cells.

4. The method of claim 1, wherein the solid support matrix (14) comprises a magnetic bead, a microfluidic channel, swab, or a well plate.

5. The method of claim 1, wherein the lysis buffer and the binding buffer are non-destructive to the intact protein and the metabolites.

6. The method of claim 1, wherein eluting the nucleic acid comprises eluting deoxyribonucleic acid and then eluting ribonucleic acid.

7. The method of claim 1, wherein eluting the nucleic acid comprises eluting ribonucleic acid and then eluting deoxyribonucleic acid.

8. The method of claim 1, wherein eluting the nucleic acid comprises co-eluting ribonucleic acid and deoxyribonucleic acid.

9. The method of claim 1, wherein the protamine conjugates (10) are configured to bind the nucleic acid having a size ranging from 25 nucleotides to greater than 15,000 nucleotides.

10. The method of claim 1, wherein eluted nucleic acid has a quantity ranging from picograms to micrograms.

11. A method, comprising:
obtaining a tissue sample; and
performing multi-analyte recovery on the tissue sample to isolate deoxyribonucleic acid, ribonucleic acid, and intact protein and metabolites from the tissue sample utilizing protamine conjugates (10) comprising protamine conjugated to a solid support matrix (14).

12. The method of claim 11, wherein the tissue sample comprises a biopsy sample, a touch imprint, human cells, non-human cells, blood, plasma, semen, saliva, or epithelial cells.

13. The method of claim 11, further comprising lysing the tissue sample in a lysis buffer prior to the multi-analyte recovery.

14. The method of claim 13, wherein performing multi-analyte recovery on the tissue sample comprises:
adding the protamine conjugates 10) to a lysate of the lysed tissue sample;
incubating the lysate in the presence of the protamine conjugates (10) in a binding buffer in a reaction mixture to bind nucleic acid to the protamine conjugates (10);
removing a supernatant from the reaction mixture containing the intact protein and metabolites; and
eluting the nucleic acid from the protamine conjugates (10).

15. The method of claim 14, wherein the lysis buffer and the binding buffer are non-destructive to the intact protein and the metabolites.
